# EUROPEAN PATENT APPLICATION

(11) **EP 4 852 014 A1**
(43) Date of publication of application: **09.12.2026**
(21) Application number: 25181150.1
(22) Date of filing: 05.06.2025
(51) Int. Cl.: G16H 40/63, G16H 40/67, G16H 30/20, G16H 40/40, H04L 69/24

(54) **MULTI-CONNECTOR SAFETY BOX**

(71) Applicant: Koninklijke Philips N.V., 5656 AE Eindhoven (NL)
(72) Inventor: WEIß, Steffen, Eindhoven (NL); VOGTMEIER, Gereon, Eindhoven (NL)
(74) Representative: Philips Intellectual Property & Standards

(57) **Abstract**

An apparatus (130) is provided for connecting one or more external devices to a medical device. The apparatus comprises a first interface (140), one or more second interfaces (150), a control circuit (160), and a certified protection arrangement (170). The control circuit (160) is configured to communicably couple to the medical device through the first interface and to communicably couple to the one or more external devices through the one or more second interfaces to control a data communication between the medical device and the one or more external devices. The certified protection arrangement (170) is configured to control at least one operating parameter of the apparatus and/or data exchanged between the medical device and the one or more external devices within a specified metric such that the medical device meets safety and performance criteria when interfacing with the apparatus.

## Description

### FIELD OF THE INVENTION

The present invention relates to an apparatus for connecting one or more external devices to a medical device and to a system comprising the apparatus.

### BACKGROUND OF THE INVENTION

Medical devices may incorporate electronic components and technology to perform their intended medical functions. These devices are designed to diagnose, monitor, treat, or manage health conditions using electronic systems. Medical devices are highly regulated because they can represent safety hazards for humans and should work effectively. Medical devices are also becoming increasingly complex with a high number of subsystems. Medical devices are also becoming increasingly interoperable, i.e., they are connected to other devices which may be medical devices and/or non-medical devices.

The design and certification of a device as medical device creates a large effort which is expected to still increase. The effort for repeating certifications after upgrading a medical device is large and should be avoided. Medical devices often have a longer lifetime than devices connected to it. Examples are external sensors connected to medical imaging systems, such as cameras or electrocardiogram (ECG)-devices. Such sensors evolve faster than the medical imaging systems, thereby requiring certifications. Additionally, ECG-devices are medical devices, but they may come from third party vendors and be exchanged. It may be a problem that the medical device must be recertified in case of exchange, change of type, or upgrades of medical or non-medical connected devices.

### SUMMARY OF THE INVENTION

Thus, there may be a need to avoid recertification of the medical device in case of exchange, change of type, and/or upgrades of medical or non-medical connected devices.

The object of the present invention is solved by the subject matter of the independent claims where further embodiments are incorporated in the dependent claims. It should be noted that the following described aspect of the invention equally applies to the apparatus and the system.

According to a first aspect of the present invention, there is provided an apparatus for connecting one or more external devices to a medical device. The apparatus comprises a first interface, one or more second interfaces, a control circuit, and a certified protection arrangement. The control circuit is configured to communicably couple to the medical device through the first interface and to communicably couple to the one or more external devices through the one or more second interfaces to control a data communication between the medical device and the one or more external devices. The certified protection arrangement is configured to control at least one operating parameter of the apparatus and/or data exchanged between the medical device and the one or more external devices within a specified metric such that the medical device meets safety and performance criteria when interfacing with the apparatus.

Accordingly, the apparatus is provided as an interface between a medical device and one or more external devices. The apparatus may also be referred to as safety box that may be a separate medical device with separate certification and switched between the medical device and the one or more external devices. The apparatus has a certified protection arrangement to control at least one operating parameter (e.g., voltage, current, power, etc.) of the apparatus and/or data (e.g., data range, data format, etc.) exchanged between the medical device and the one or more external devices within a specified metric such that the medical device meets safety and performance criteria when interfacing with the apparatus. As the protection arrangement is certified separately from the medical device, it possible to avoid recertification of the medical device in case of major changes and/or upgrades of the external device(s). Such changes may maximally require redesign and recertification of the apparatus instead of the medical device.

The apparatus may be implemented as a stand-alone device with connectors to the medical device and to the connected external device(s). The apparatus may alternatively by loosely physically integrated into the medical device, such that looks as if it was a part of the medical device. In any case, the apparatus is a separate medical device that is certified separately from the medical device, which avoids recertification of the medical device in case of major changes and/or upgrades of the external device (s).

According to an embodiment of the present invention, the certified protection arrangement comprises one or more of: a radio frequency (RF) limiter, an electrical isolation arrangement, an overvoltage protection arrangement, an overcurrent protection arrangement, and a connect-disconnect protection arrangement.

These exemplary certified protection arrangements may be used to control at least one operating parameter (e.g., amplitude of an RF signal, current, voltage, etc.) of the apparatus within a specified metric.

According to an embodiment of the present invention, the certified protection arrangement comprises a data plausibility checking circuit configured to perform a plausibility check of the data exchanged between the medical device and the one or more external devices; and provide data that passes the plausibility check to the medical device.

The plausibility check is a process used to verify the credibility and logical consistency of data, inputs, and/or system states. For example, the data plausibility check circuit may check all data inflow and only allow "defined and certified" data flow output to the medical device.

According to an embodiment of the present invention, the data plausibility checking circuit is further configured to replace data that fails to pass the plausibility check with uncritical data.

For example, the data plausibility checking circuit may buffer the data flow to and from the medical device, check and then finally send data with a label "data format proof". Any missing data may be filled with uncritical data.

According to an embodiment of the present invention, the plausibility check comprises one or more of the following: consistency verification, range and threshold check, data format and structure validation, error detection and correction, contextual relevance, and timing check and check for fulfilling a communication speed requirement.

According to an embodiment of the present invention, the certified protection arrangement comprises a self-diagnosis circuit configured to generate interface status information indicating a condition and connecting status of the one or more second interfaces, and communicate the generated interface status information to the medical device.

The self-diagnosis circuit may ensure proper functionality and give status update information to the medical device including the information which signal from external source to medical device is connected and will deliver signals and/or data.

According to an embodiment of the present invention, for at least one external device that is currently disconnected, the self-diagnosis circuit is configured to send a defined signal to the medical device that is uncritical and that is configured to indicate that the at least one external device is not connected.

For example, for sensors that are currently disconnected, the apparatus may send a defined signal to the medical device that is uncritical but also shows that no sensor is connected. Transient signals are reshaped to uncritical signal levels.

According to an embodiment of the present invention, the certified protection arrangement is configured to be tailored a hazard originating from the one or more external devices.

For example, the apparatus may be implemented to provide a protection mechanism that are tailored to the hazards originating from the external device. This may not only externalize the effort to protect the medical device to the apparatus, but the effort will become smaller since tailored. If new hazards arise from the external device(s), only the safety measures of the apparatus must be adapted, and the medical device can stay as is.

According to an embodiment of the present invention, the first interface comprises a vendor-specific interface.

According to an embodiment of the present invention, the apparatus is a stand-alone device, or a device that is physically integrated into the medical device.

According to a second aspect of the present invention, there is provided a system. The system comprises a medical device, one or more external devices, and the apparatus according to the first aspect for connecting the medical device to the one or more external devices, wherein the apparatus is certified separately from the medical device.

According to an embodiment of the present invention, the medical device comprises a medical imaging system.

According to an embodiment of the present invention, the one or more external devices comprise a non-medical device.

According to an embodiment of the present invention, the one or more external devices comprise a medical device.

According to an embodiment of the present invention, the one or more external devices include one or more sensor devices.

These and other aspects of the present invention will become apparent from and be elucidated with reference to the embodiments described hereinafter.

### BRIEF DESCRIPTION OF THE DRAWINGS

These and other aspects of the invention will be apparent from and elucidated further with reference to the embodiments described by way of examples in the following description and with reference to the accompanying drawings, in which
Fig. 1 schematically depicts an exemplary system for implementing embodiments of the present disclosure.
Fig. 2 schematically depicts an exemplary apparatus for implementing embodiments of the present disclosure.
Fig. 3 illustrates an exemplary certified protection arrangement for implementing embodiments of the present disclosure.

It should be noted that the figures are purely diagrammatic and not drawn to scale. In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

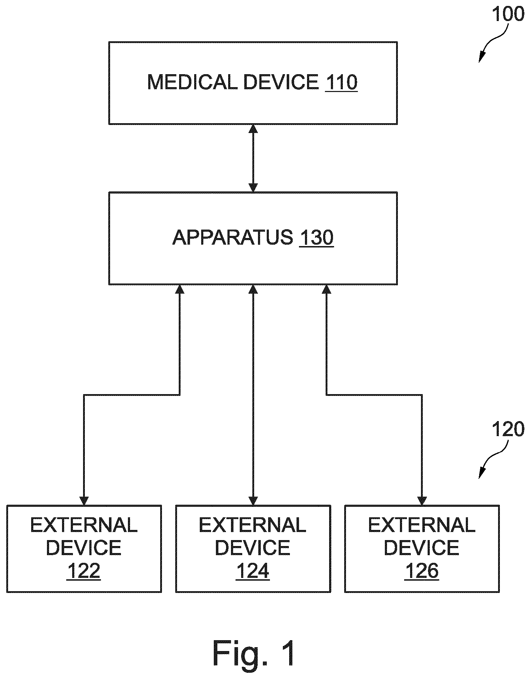

Fig. 1 illustrates an exemplary system 100 according to an embodiment of the present invention. System 100 comprises a medical device 110, one or more external devices 120, and an apparatus 130 for connecting the one or more external devices 120 to the medical device 110.

The medical device 110 may be a piece of equipment used in the healthcare sector that incorporates electronic components to perform its functions. The medical device 110 may be a device that diagnoses, monitors, or treats medical conditions and may range from simple instruments to complex systems. In some examples, the medical device 110 may be an interoperable medical device that is designed to work seamlessly with other devices or systems, facilitating the exchange and use of data to improve healthcare delivery. As an example, the medical device 110 may be an interoperable medical imaging system are tools in healthcare, providing detailed images of the body's internal structures to aid in diagnosis and treatment planning. Examples of the interoperable medical imaging system, provided in a non-restrictive list for purposes of illustration only, may include e.g., magnetic resonance imaging (MRI) systems, computed tomography (CT) scanners, ultrasound machines, X-ray systems, positron emission tomography (PET) scanners, digital mammography systems, fluoroscopy systems, single photon emission computed tomography (SPECT) systems.

The medical device 110 often connects to one or more external devices 120 to enhance functionality, facilitate data sharing, and improve patient care. For instance, the medical device 120 may comprise one or more device interfaces (not shown) configured to connect the medical device 110 to one or more external devices and/or systems and enabling exchange of data between the medical device and the one or more external devices and/or systems.

The one or more external devices 120 may include hardware, firmware and/or software. Fig. 1 illustrates three exemplary external devices 122, 124 and 126 which may include one or more of the exemplary external devices described below.

In some examples, the one or more external devices 120 may include one or more patient monitoring sensors that are devices used to continuously or periodically measure various physiological parameters of a patient. These sensors are integral to modern healthcare, providing real-time data that helps in the assessment and management of a patient's health status. Examples of the patient monitoring sensors may include, but are not limited to, ECG-sensors for recording the electrical activity of the heart, respiratory rate sensors for tracking the number of breaths per minute, often integrated into multiparameter monitoring systems, blood pressure sensors for monitoring systolic and diastolic blood pressure, pulse oximeters for measuring the oxygen saturation level in the blood and pulse rate, typically clipped onto a finger or earlobe, and motion sensors for detecting movement and activity levels, used in fall detection systems or rehabilitation monitoring.

In some examples, the one or more external devices 120 may include a picture Archiving and Communication Systems (PACS). For instance, medical imaging systems frequently connect to PACS to store, retrieve, and share medical images. PACS allows healthcare providers to access images from different locations, facilitating collaborative diagnosis and treatment planning.

In some examples, the one or more external devices 120 may include workstations and Viewing Software. For instance, some medical imaging systems may connect to specialized workstations equipped with software for viewing, analyzing, and interpreting medical images. These workstations often have advanced tools for image manipulation and enhancement.

In some examples, the one or more external devices 120 may include mobile devices and Apps. For instance, some medical imaging systems may offer connectivity with mobile devices, enabling healthcare providers to view images on tablets or smartphones, which can be useful in emergency or remote settings.

In some examples, the one or more external devices 120 may include electronic health record (EHR) systems. Integration with EHR systems may enable imaging data to be included in a patient's comprehensive health record, allowing for better tracking of patient history and facilitating informed decision-making.

In some further examples, the one or more external devices 120 may include diagnostic and analysis tools. For example, some imaging systems can connect to software tools that provide automated analysis, such as detecting anomalies or quantifying measurements, which assists radiologists in their evaluations.

The design and certification of the medical device 110 may create a large effort which is expected to still increase. The effort for repeating certification after upgrading a device is large and should be avoided. Medical devices may often have a longer lifetime than some of the external devices connected to them. For instance, external sensors, such as cameras or ECG-sensors may evolve faster than the medical device. Some of the external devices may come from third party vendors and may be exchanged. It is a problem that the medical device must be recertified in case of exchange, change of type, or upgrades of medical or non-medical connected external devices.

Towards this end, the apparatus 130 is provided for connecting one or more external devices 120 to the medical device 110. The apparatus 130 may also be referred to as a safety box, which has a certified protection mechanism aimed at preventing damage to or interference with the medical device110. The apparatus 130 may be implemented as a stand-alone device with connectors to the medical device 110 and to the one or more connected external devices 120. The apparatus 130 may alternatively be loosely physically integrated into the medical device 110, such that the apparatus 130 looks as if it is a part of the medical device. In any case, the apparatus 130 may be implemented as a separate medical device that is certified separately from the medical device, thereby avoiding recertification of the medical device in case of major changes and/or upgrades of the external device(s).

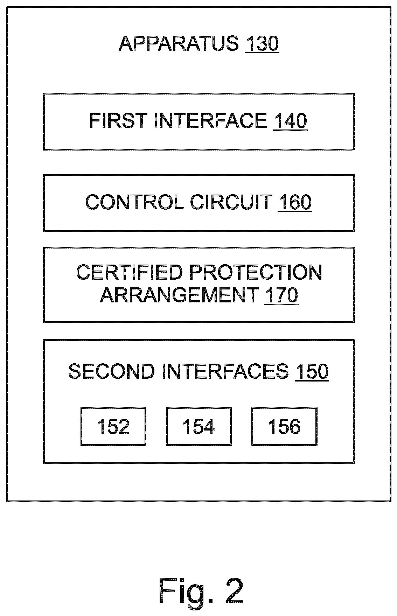

Fig. 2 illustrates an exemplary apparatus 130 for connecting one or more external devices 120, such as the external devices 122, 124 and 126 shown in Fig. 1 to the medical device 110. The apparatus 130 comprises a first interface 140, one or more second interfaces 150, a control circuit 160, and a certified protection arrangement 170. As an example, Fig. 2 illustrates three exemplary second interfaces 152, 154 and 156, through which the apparatus 130 may be communicatively coupled to the external devices 122, 124 and 126 shown in Fig. 1.

The first interface 140 may is an interface between the apparatus 130 and the medical device 110 that facilitates the communication and the exchange of data between the two entities. This interface may involve various technologies and protocols to ensure accurate data transmission, real-time monitoring, and integration with broader healthcare systems. In some implementations, the first interface may comprise a vendor-specific interface.

In some implementations, the first interface 140 may be configured to support wired protocols, such as USB, RS-232, or Ethernet. The first interface 140 may alternatively or additionally support wireless protocols, such as Bluetooth, Wi-Fi, Zigbee, or NFC are used for wireless communication.

In some implementations, the first interface 140 may be configured to support standardized formats, such as Health Level Seven (HL7), Digital Imaging and Communications in Medicine (DICOM), and as Fast Healthcare Interoperability Resources (FHIR), to ensure compatibility and interoperability.

In some implementations, the first interface 140 may be configured to support real-time data transfer to support real-time data transfer, crucial for applications requiring immediate feedback, such as continuous glucose monitoring or cardiac monitoring. In some implementations, the first interface 140 may be configured to support batch data transfer. For example, data may be collected over time and transmitted in batches, suitable for non-critical applications.

In some implementations, the first interface 140 may support data encryption to ensure that data transmitted between the apparatus and the medical device is secure and protected against unauthorized access, and comply with relevant healthcare regulations and standards, such as HIPAA in the U.S. and GDPR in Europe.

The one or more second interfaces 150 are interface(s) between the apparatus 130 and the one or more external devices 120 that facilitates the communication and exchange of data between the two entities. This interface can involve various technologies and protocols to ensure accurate data transmission, real-time monitoring, and integration with broader healthcare systems.

In some implementations, some of the one or more second interfaces 150 may support wired protocols. These include interfaces like USB, RS-232, or Ethernet, which provide reliable and secure data transmission between the external device(s) 120, such as sensors, and the apparatus 130. In some implementations, some of the one or more second interfaces 150 may support wireless protocols. For example, technologies such as Bluetooth, Wi-Fi, Zigbee, or NFC are used for wireless communication, offering flexibility and mobility, especially in wearable sensors.

In some implementations, some of the one or more second interfaces 150 may support standardized data formats. Use of standardized data formats may ensure compatibility and interoperability. Common standards may include HL7 for health data exchange and IEEE 11073 for personal health devices. In some implementations, some of the one or more second interfaces 150 may support proprietary data formats. For example, some external devices 120 may use proprietary data formats, requiring specific software or middleware for data interpretation and integration.

In some implementations, some of the one or more second interfaces 150 may support analog to digital conversion. For example, sensors often collect analog signals that need to be converted into digital data for processing and analysis by the medical device. In some implementations, some of the one or more second interfaces 150 may support filtering and calibration for filtering noise and calibrating sensor data to ensure accuracy and reliability.

In certain implementations, some of the one or more second interfaces 150 may be set up to facilitate real-time data transfer, which is essential for applications that demand immediate feedback, such as continuous glucose monitoring or cardiac monitoring. In certain implementations, some of the one or more second interfaces 150 may be configured to enable batch data transfer, where data is accumulated over time and transmitted in batches, making it suitable for non-critical applications.

In certain implementations, some of the one or more second interfaces 150 may be configured to support network integration that enables connection to hospital networks, allowing for seamless data transfer to EHRs and other systems.

In certain implementations, some of the one or more second interfaces 150 may be configured to support integration with other medical devices and healthcare systems, facilitating comprehensive patient monitoring and data analysis. In certain implementations, some of the one or more second interfaces 150 may be configured to ensure that data transmitted between the sensor and the apparatus is secure and protected against unauthorized access and comply with healthcare regulations and standards to ensure patient safety and data security, such as HIPAA in the U.S. and GDPR in Europe.

The control circuit 160 is configured to communicably couple to the medical device 110 through the first interface 140 and to communicably couple to the one or more external devices 120 through the one or more second interfaces 150 to control a data communication between the medical device and the one or more external devices. The control circuit 160 may include discrete and/or integrated circuitry, application ASIC, a SOC, and combinations thereof.

The certified protection arrangement 170 is configured to control at least one operating parameter of the apparatus and/or data exchanged between the medical device and the one or more external devices within a specified metric such that the medical device meets safety and performance criteria when interfacing with the apparatus. The operating parameter(s) that may be controlled include, for example, voltage, current, amplitude of an RF signal, and similar factors. The data exchange between the medical device and the one or more external devices that may be controlled include, for example, a range of data, data formats, data consistency, data errors, and similar factors.

The certified protection arrangement 170 has a medical device certification and complies with regulations set by governmental bodies, such as the U.S. Food and Drug Administration (FDA), the European Medicines Agency (EMA), or other national health authorities, as well as international standards such as IEC 60601 (safety standards for medical electrical equipment). The certified protection arrangement 170 is certified separately from the medical device. In this way, external noncertified devices may be connected to the medical device without the need for certification.

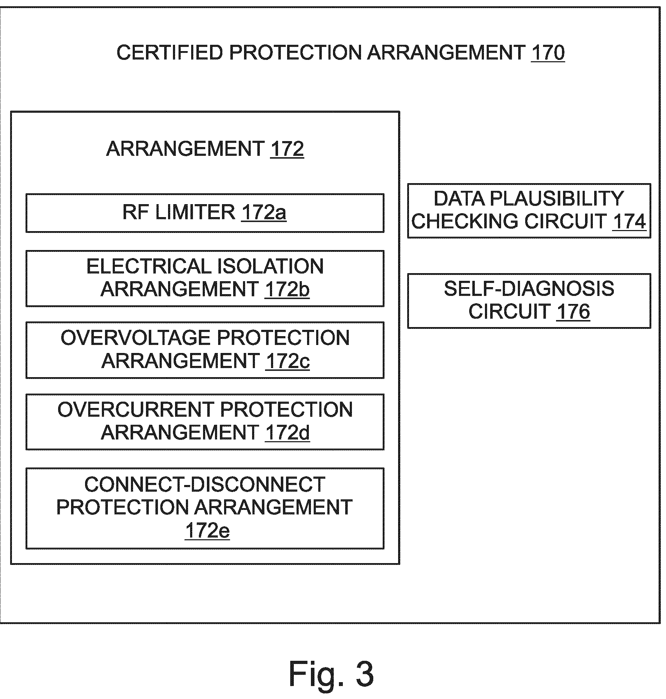

Fig. 3 illustrates an exemplary certified protection arrangement 170 that comprises a plurality of exemplary arrangements 172 to control at least one operating parameter of the apparatus within a specified metric. As shown, the plurality of exemplary arrangements includes one or more of an RF limiter 172a, an electrical isolation arrangement 172b, an overvoltage protection arrangement 172c, an overcurrent protection arrangement 172d, and a connect-disconnect protection arrangement 172e.

The RF limiter 172a is a device used in RF circuits to protect sensitive components from high-power signals that could potentially cause damage. It acts as a safeguard by limiting the amplitude of incoming RF signals to a specified level, ensuring that the components downstream are not exposed to excessive power levels. In some examples, the RF limiter 172a may include a passive limiter which utilizes diodes or other passive components to limit signal power. These are typically simpler and more cost-effective but may introduce some signal distortion. In some examples, the RF limiter 172a may include an active limiter that uses active components such as transistors to dynamically control signal levels. These can offer more precise control but are generally more complex and expensive. In some examples, the RF limiter 172a may include a combination of a passive limiter and an active limiter. The RF limiter may be used in the apparatus 130 in conjunction with a medical device 110 (such as medical imaging systems) that employs RF technology. RF limiters 172a may be beneficial for preventing damage to expensive and sensitive RF components in the medical device 110, extending their lifespan and reliability. By controlling signal levels, RF limiters 172a may help maintain the integrity of RF signals, reducing the risk of distortion and ensuring accurate signal processing. They contribute to the overall reliability of RF systems by preventing failures due to excessive signal power.

The electrical isolation arrangement 172b is used for separating electrical circuits to prevent current flow between them, ensuring that each circuit operates independently without interference or risk of electrical shock. For example, the electrical isolation arrangement 172b may comprise a transformer that uses electromagnetic induction to transfer electrical energy between circuits while maintaining physical separation. For example, the electrical isolation arrangement 172b may comprise a capacitive isolation arrangement that uses capacitors to block DC signals while allowing AC signals to pass, providing isolation in certain applications. For example, the electrical isolation arrangement 172b may comprise an optoisolator that uses light to transfer signals between circuits, providing electrical isolation by converting electrical signals to light and back to electrical signals. The electrical isolation arrangement 172b may prevent electrical shock by ensuring that high-voltage circuits do not come into contact with low-voltage circuits or users. The electrical isolation arrangement 172b may reduce noise and interference between circuits, maintaining the integrity of signals in sensitive applications. The electrical isolation arrangement 172b may ensure patient safety by isolating electrical circuits from the patient interface.

The overvoltage protection arrangement 172c may be designed to safeguard electrical and electronic devices from voltage levels that exceed their normal operating range. For example, the overvoltage protection arrangement 172c may include a circuit breaker and fuse which automatically disconnects the power supply when an overvoltage condition is detected, preventing damage to the medical device 110. For example, the overvoltage protection arrangement 172c may comprise a transient voltage suppression (TVS) diode that absorbs and dissipates transient overvoltages, protecting sensitive electronic components of the medical device 110. For example, the overvoltage protection arrangement 172c may comprise an uninterruptible power supply (UPS) that provides backup power and voltage regulation, ensuring continuous operation and protection against overvoltage conditions. Overvoltage can occur due to various reasons, such as power surges, lightning strikes, or faults in the electrical grid. This overvoltage protection arrangement 172c may help prevent damage to sensitive components of the medical device 110 and ensure patient safety and equipment reliability.

The overcurrent protection arrangement 172d is designed to prevent excessive current flow in electrical circuits, which can lead to overheating, damage to components, and potential fire hazards. For example, the overcurrent protection arrangement 172d may comprise a fuse that contains a metal wire or strip that melts when the current exceeds a certain level, breaking the circuit and stopping the flow of electricity. For example, the overcurrent protection arrangement 172d may comprise a current limiter that restricts the maximum current that can flow through a circuit, protecting sensitive components of the medical device from damage. For example, the overcurrent protection arrangement 172d may comprise an active component, such as a circuit breaker that automatically disconnect the circuit when an overcurrent is detected and can be reset once the issue is resolve. By preventing damage from excessive current, the overcurrent protection arrangement 172d may help extend the lifespan of electrical components and reduce maintenance costs of the medical device 110.

The connect-disconnect protection arrangement 172e is used to safely manage the connection and disconnection of electrical circuits and devices. This type of protection is crucial for preventing damage to equipment, ensuring user safety, and maintaining the reliability of electrical systems. For example, the connect-disconnect protection arrangement 172e may comprise a circuit breaker that automatically disconnects circuits when they detect fault conditions such as overcurrent or short circuits. They can be manually reset once the issue is resolved. For example, the connect-disconnect protection arrangement may comprise a relay, which is an electromechanical or solid-state device that controls the connection and disconnection of the one or more external devices 120 based on specific conditions, such as voltage or current levels. In some examples, the connect-disconnect protection arrangement 172e may comprise an interlock, which is a safety mechanism that prevents the connection or disconnection of the one or more external devices unless certain conditions are met, ensuring safe operation. The connect-disconnect protection arrangement 172e may ensure that the one or more external devices can be connected or disconnected without causing harm to the medical device 110. The connect-disconnect protection arrangement 172e may quickly disconnect faulty external device(s) to prevent damage to the medical device 110 and maintain system integrity. The connect-disconnect protection arrangement 172e may allow for the safe connection and disconnection of loads to prevent overloading and ensure balanced power distribution. The connect-disconnect protection arrangement 172e may also ensure that the one or more external devices can be connected or disconnected without affecting data integrity and continuity. This may be particularly beneficial where reliable data transmission is critical, such as medical imaging systems. For example, the connect-disconnect protection arrangement 172e may implement buffers to temporarily store data during transmission interruptions to ensure that no data is lost when connectivity is restored. For example, the connect-disconnect protection arrangement 172e may ensure that data is synchronized across devices and systems after reconnection, which may help prevent incomplete datasets and maintain consistency.

In some implementations, the certified protection arrangement 170 may be configured to control data exchanged between the medical device and the one or more external devices within a specified metric. For example, as shown in Fig. 3, the certified protection arrangement 170 may comprise a data plausibility checking circuit 174 configured to perform a plausibility check of the data exchanged between the medical device and the one or more external devices. The data plausibility checking circuit 174 may include discrete and/or integrated circuitry, application-specific integrated circuit (ASIC), a system on chip (SOC), and combinations thereof. In some implementations, the data plausibility checking circuit 174 may be part of the control circuit 160. The plausibility check is a process used to verify the credibility and logical consistency of data, inputs, and/or system states. For example, the data plausibility checking circuit 174 may be configured to perform one or more of the plausibility checks exemplified below.

In some implementations, the plausibility check may include consistency verification to evaluate whether related data points are logically consistent with each other. For instance, the consistency verification may include time-series analysis to analyze data over time to ensure consistency in trends and patterns. Sudden spikes or drops may indicate errors. For example, the consistency verification may include synchronization check to ensure that data timestamps are consistent and synchronized across different sensors, especially in systems with multiple data sources. For example, the consistency verification may include cross-referencing to compare data against other related datasets or know benchmarks to ensure consistency.

In some implementations, the plausibility check may include a range and threshold check that ensures data values fall within predefined acceptable ranges. For example, the range and threshold check may ensure that sensor readings fall within expected and predefined ranges. Values outside these ranges may indicate sensor malfunction or erroneous data.

In some implementations, the plausibility check may include data format and structure validation to ensure that data adheres to expected formats required by the medical device, such as date formats or numerical precision, such as numerical values, timestamps, or specific units of measurement. The data format and structure validation may also verify that data structures, such as tables or records, are complete and correctly organized.

In some implementations, the plausibility check may include error detection and correction. For example, the error detection may implement error codes or flags that indicate potential issues with sensor readings. For example, the error correction may implement algorithms to automatically correct minor errors or inconsistencies in sensor data.

In some implementations, the plausibility check may include contextual relevance to evaluate whether data makes sense within its specific context or application. For example, a medical device reading should align with expected physiological parameters.

In some implementations, the plausibility check may include timing check and check for fulfilling a communication speed requirement. For example, medical imaging systems often generate large volumes of data, especially with high-resolution images such as MRI or CT scans. The communication speed requirement would assess whether the system can transmit these images efficiently over networks, whether local or remote, without significant delays. In such cases, the apparatus is preferred to be capable of processing images quickly to facilitate timely diagnosis. This plausibility check may ensure that the medical imaging system can operate efficiently and effectively in clinical settings, providing timely and accurate information to the external device(s).

The data plausibility checking circuit 174 may be configured to provide data that passes the plausibility check to the medical device. For example, the data plausibility check circuit 174 may check all data inflow and only allow "defined and certified" data flow output to the medical device.

The data plausibility checking circuit 174 may be further configured to replace data that fails to pass the plausibility check with uncritical data. Uncritical data for a medical device may refer to information that, while useful, does not directly impact the safety, efficacy, or essential functioning of the device. This type of data is typically not vital for the immediate operation or decision-making processes related to patient care. For example, the data plausibility checking circuit 174 may buffer the data flow to and from the medical device, check and then finally send data with a label "data format proof". Any missing data may be filled with uncritical data.

In some implementations, as shown in Fig. 3, the certified protection arrangement 170 may comprise a self-diagnosis circuit 176 configured to generate interface status information indicating a condition and connecting status of the one or more second interfaces and communicate the generated interface status information to the medical device. For example, one or more external devices 120 may be connected to the apparatus 130 in a plug and play manner. Plug-and-play connectivity can be achieved through Universal Serial Bus (USB), FireWire, or other hot plug/hot swap connections, for instance. The self-diagnosis circuit 176 may provide a framework to execute device drivers that gather information from one or more connected external devices 120. Information may be collected from and transmitted to one or more external devices 120 via the one or more second interfaces 150, such as one featuring a USB connector/converter, enabling the one or more devices 120 to communicate with the apparatus 130. In some examples, the self-diagnosis circuit 176 may use the one or more second interfaces 150 and a driver library to detect one or more external devices 120, being connected or disconnected from the apparatus 130. The device library may include device drivers for a plurality of external devices 120. In some implementations, for at least one external device that is currently disconnected, the self-diagnosis circuit 176 may be configured to send a defined signal to the medical device that is uncritical and that is configured to indicate that the at least one external device is not connected. For example, for sensors that are currently disconnected, the self-diagnosis circuit 176 may send a defined signal to the medical device that is uncritical but also shows that no sensor is connected. Transient signals may be reshaped to uncritical signal levels. In this way, the self-diagnosis circuit 176 may ensure proper functionality and give status update information to the medical device, including e.g., the information which signal from external source to the medical device is connected and will deliver signals and/or data. The self-diagnosis circuit 176 may include discrete and/or integrated circuitry, an ASIC, an SOC, and combinations thereof. In some implementations, the self-diagnosis circuit 176 may be part of the control circuit 160.

In some implementations, as shown in Fig. 3, the certified protection arrangement is configured to be tailored to a hazard originating from one or more external devices. Hazards from the external device(s) 120 for the medical device 110 may arise from various factors that may compromise the safety, functionality, or accuracy of the medical device 110. For example, some external devices 120 may emit electromagnetic fields that may interfere with the operation of the apparatus, which may lead to distorted image, inaccurate readings, or malfunctioning equipment, potentially compromising diagnostic accuracy. In such case, the certified protection arrangement may be tailored to comprise an electromagnetic shield mechanism to block or reduce electromagnetic fields and interference from affecting sensitive electronic equipment. For example, non-medical devices connected to the medical device, such as computers or network devices, may introduce vulnerabilities that can be exploited by cyber threats. Unauthorized access or data breaches can compromise patient privacy and the integrity of medical data. Accordingly, the certified protection arrangement may be tailored to implement robust cybersecurity measures. In this way, the certified protection arrangement may be implemented to provide only such precautions that are tailored to the hazards originating from the external device. This may not only externalize the effort to protect the medical device to the apparatus, but the effort will become smaller since tailored. If new hazards arise from the external device, only the safety measures of the apparatus must be adapted, and the medical device can stay as is.

Some or all components of the apparatus may be arranged in hardware such as a suitably programmed FPGA or as hardwired IC chip. One or more features of the apparatus disclosed herein may be configured or implemented as/with circuitry encoded within a computer-readable medium, and/or combinations thereof. Circuitry may include discrete and/or integrated circuitry, application ASIC, an SOC, and combinations thereof., a machine, a computer system, a processor and memory, a computer program. Some or all components of the apparatus described herein may be implemented using instructions, code, and/or other software and/or firmware, etc. stored on a machine accessible or readable medium and executable by, for example, a processor system. When any of the appended claims are read to cover purely software and/or firmware implementation, at least one of the components is hereby expressly defined to include a tangible medium such as a memory, DVD, CD, etc. storing the software and/or firmware.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An apparatus (130) for connecting one or more external devices to a medical device, the apparatus comprising:
- a first interface (140);
- one or more second interfaces (150);
- a control circuit (160), configured to communicably couple to the medical device through the first interface and to communicably couple to the one or more external devices through the one or more second interfaces to control a data communication between the medical device and the one or more external devices; and
- a certified protection arrangement (170) configured to control at least one operating parameter of the apparatus and/or data exchanged between the medical device and the one or more external devices within a specified metric such that the medical device meets safety and performance criteria when interfacing with the apparatus.

2. The apparatus according to claim 1,
wherein the certified protection arrangement comprises one or more of:
- a radio frequency, RF, limiter (172a);
- an electrical isolation arrangement (172b);
- an overvoltage protection arrangement (172c);
- an overcurrent protection arrangement (172d); and
- a connect-disconnect protection arrangement (172e).

3. The apparatus according to claim 1 or 2,
wherein the certified protection arrangement comprises a data plausibility checking circuit (174) configured to:
- perform a plausibility check of the data exchanged between the medical device and the one or more external devices; and
- provide data that passes the plausibility check to the medical device.

4. The apparatus according to claim 3,
wherein the data plausibility checking circuit is further configured to replace data that fails to pass the plausibility check with uncritical data.

5. The apparatus according to claim 3 or 4,
wherein the plausibility check comprises one or more of the following:
- consistency verification;
- range and threshold check;
- data format and structure validation;
- error detection and correction;
- contextual relevance; and
- timing check and check for fulfilling a communication speed requirement.

6. The apparatus according to any one of the preceding claims,
wherein the certified protection arrangement comprises a self-diagnosis circuit (176) configured to:
- generate interface status information indicating a condition and connecting status of the one or more second interfaces; and
- communicate the generated interface status information to the medical device.

7. The apparatus according to claim 6,
wherein for at least one external device that is currently disconnected, the self-diagnosis circuit is configured to send a defined signal to the medical device that is uncritical and that is configured to indicate that the at least one external device is not connected.

8. The apparatus according to any one of the preceding claims,
wherein the certified protection arrangement is configured to be tailored a hazard originating from the one or more external devices.

9. The apparatus according to any one of the preceding claims,
wherein the first interface comprises a vendor-specific interface.

10. The apparatus according to any one of the preceding claims,
wherein the apparatus is a stand-alone device, or a device that is physically integrated into the medical device.

11. A system (100), comprising:
- a medical device (110);
- one or more external devices (120); and
- the apparatus (130) according to any one of the preceding claims for connecting the medical device to the one or more external devices, wherein the apparatus is certified separately from the medical device.

12. The system according to claim 11,
wherein the medical device comprises a medical imaging system.

13. The system according to claim 11 or 12,
wherein the one or more external devices comprise a non-medical device.

14. The system according to any one of claims 11 to 13,
wherein the one or more external devices comprise a medical device.

15. The system according to claim 14,
wherein the one or more external devices include one or more sensor devices.
